# EUROPEAN PATENT SPECIFICATION

(11) **EP 1 795 216 B1**
(45) Date of publication and mention of the grant of the patent: **28.05.2008**
(21) Application number: 05380265.8
(22) Date of filing: 05.12.2005
(51) Int. Cl.: A61M 1/00

(54) **Medical suction apparatus**
Medizinisches Absauggerät
Appareil d'aspiration médical

(43) Date of publication of application: 13.06.2007
(73) Proprietor: Hersill, S.L., 28935 Mostoles Madrid (ES)
(72) Inventor: Martinez Jordan, Carlos, 28022 MADRID (ES); Martinez Llanos, David Angel, 28007 MADRID (ES)
(74) Representative: Esteban Perez-Serrano, Maria Isabel

(56) References cited:
- EP-A- 0 655 253
- DE-A1- 1 952 065
- US-A- 4 112 949
- US-A- 5 934 888
- US-B1- 6 652 484

## Description

The present invention relates to a medical suction apparatus designed to be driven by the user by means of the foot or, failing that, with the hand.

The medical suction apparatuses are used with the intention of applying vacuum for the suction of liquid fluids, or solids suspended in liquids, from an organ or region located of the patient to a collection tank. The necessity or prescription to aspire liquids from the patient is usual in different situations or interventions within the medical practice, these applications attend to the following classification: suction of the air-way in patients with respiratory trauma and patients with assisted ventilation, endoscopic suction, suction of the surgical field, obstetrical suction, abdominal drainage, toracic drainage, breastmilk suction and, finally, the application as a support to an obstetrical suction cup (assistance to the birth). Each intervention requires a specific combination of two physical magnitudes: the negative pressure applied (vacuum) and the suction flow.

Ever since medical science describes the first necessities of application of vacuum to the patient, medical succion apparatuses have been developed to be driven by the user by means of the foot or the hand; without forgetting the old utensils of generic application described by GB190420358 in 1905, recently and coexisting with the modern electrical suction apparatuses, it has been designed apparatuses looking for simplicity and normally focused to the use in some specific of the previously described application, these apparatuses are identified next: US6652484B1 defines a pump for the suction of maternal milk that it is collected in a tank connected with a detachable piston pump driven by the foot; it is a mere adaptation of a generic pumping apparatus connected to a tank by means of a hose. For the same application that the previous one is the design US6110140, more compact and in this case driven with a hand. For the applications of toracic and abdominal drainage, that need very low magnitudes of vacuum and volume, it has been designed the apparatus US4112949, whose assembly has two sets cylinder-piston with the cylinders anchored to the rigid half of the back-bone; the other half is elastic and allows to move the cylinders by means of the user's force, the operation is based on which when reducing the chamber of the cylinders, these will tend to their position of rest, and if these cylinders are in contact with the fluids of the patient, these will be absorbed towards the apparatus; when the cylinders arrive at their rest position the deposit is already full, and will need to be disassembled and drained by the user to make a new cycle of operation. The apparatus US4699190 is apt for several sucction applications: air-way, endoscopic and surgical; it is made up of a complex assembly that contains a cylinder whose piston is driven by a pedal through a handle, for which the cylinder admits the rotation on an axis parallel to its base, the communication between the cylinder and the deposit is by means of elastic hoses, being all the components assembled on an external frame that supports all the mechanisms. Finally, the apparatus US5934888 uses a mechanism of pedal in balance beam, thus obtaining to alternate the suction in the two contiguous chambers, obtaining a continuous high flow of suction, advantage that is added to being an apparatus of great constructive simplicity due to the lack of springs; on the other hand, its great disadvantage is the cleaning difficulty, as it has not cylinders, the sealing with the pistons is made by means of bellows located in the own collection tank, which implies the high probability that the residuals of liquids can obstruct the valves or can be lodged in the bellows.
For applications noncovered by the previous known apparatuses or for users who require greater and better benefits as: higher capacity of the tank, vacuum indicator (sensor), vacuum regulation system, mechanism of bacterial filtration and overfill safety device; at the moment, the only known option are the electrical suction apparatuses; but these also present some disadvantages: limited autonomy (access to the mains or life of the batteries), high volume and weight (contain at least electrical motor, transformer and batteries), high price and, finally, the risk of making the apparatus useless due to an accidental contamination of the mechanisms, since they do not support sterilization (temperature greater than 120°C in humid atmosphere and high pressure).

The object of the present invention is to provide a medical suction apparatus designed to be driven by means of the foot or, failing that, with the hand, that, maintaining the benefits of the apparatuses actually known (driving by the user, no need of electrical energy, small volume, reduced weight and low cost), reaches in addition the usual benefits of the electrical apparatuses (high levels of vacuum and flow, great capacity of the collection tank, vacuum sensor, vacuum regulation system, bacterial filter and overfill safety device), and contributing with the addition of new benefits: better ergonomics, full sterilization capacity (autoclave and gamma radiation), simplicity of assembly (reduced number of parts), repairing easily (assembled by a user without technical knowledge) and capacity to integrate a secondary collection tank (necessary for the application of suction of the surgical field).

This way the apparatus is apt for all the situations or interventions described in the initial classification.

By means of the present invention it is considered the construction of an apparatus, designed to work supported on a horizontal surface, that has a pedal in its upper part together with the axis of a piston of vertical displacement; interposed between the pedal and the piston there is a cylinder assembled with its inside surface as stroke for the basis of the piston; the cylinder-piston set will be assembled on a tank that at the same times acts as liquids collection tank, serves as supporting frame for the apparatus and serves as anchorage for the cylinder-piston set.
When exerting a descendent vertical force on the pedal (suction phase), that is rigidily assembled to the axis of the piston, it is reduced the pressure (vacuum) in the chamber of the cylinder, due to the watertightness produced by the segments lodged in the basis of the piston and by the ring seal located in the neck of the cylinder; the vacuum generated maintains closed the expulsion valve and causes the opening of the suction valve, allowing therefore the air flow from the tank towards the cylinder, which implies the reduction of the pressure in the tank (vacuum); the physical union between the cylinder and the tank is made by means of a tube bent into an elbow, 90° downwards, part of the own cylinder, that will be coupled in its corresponding orifice of the tank during the ancorage of the cylinder-piston set; this tube also serves as support of the vacuum sensor, the relief valve and the blocking system of the pedal; the vacuum generated in the tank will be applied directly to the patient through a suction catheter connected to the patient connection of the tank.
The vertical going up of the piston or the pedal (recovery phase) takes place thanks to the potential energy accumulated in a spring lodged in the inside of the hollow axis of the piston, and caused by the compression that in this spring produces a vertical rod fixed to the tank; so that this interaction can take place, it is necessary that the cylinder-piston set is assembled on its specific anchorage of the tank, position in which the mentioned vertical rod is inside the hollow axis of the piston; when beginning the recovery phase it is generated, inside the cylinder chamber, a pressure superior to the tank one, which causes the immediate closing of the suction valve, avoiding therefore the loss of vacuum from the tank; when the pressure in the cylinder surpasses to the atmospheric pressure, it takes place the opening of the expulsion valve.
A suction phase followed by a recovery phase compose an operation cycle of the apparatus. Thanks to that the suction valve stays closed during the recovery phase and the expulsion valve stays closed during the suction phase, the vacuum in the tank is increased cycle after cycle.
A mechanism, secondary or accessory for the main mechanism of operation but that is also a fundamental part of the invention, is the set vacuun sensor + relief valve + blocking system of the pedal; it is a device based on the longitudinal displacement of a rod throughout a watertight manifold connected to the tank, and for his location it makes use of the horizontal section of the tube bent into an elbow, part of the own cylinder, previously described, that links the cylinder chamber with the tank. The operation as vacuum sensor is reached by locating a spring in the inner of the horizontal section of the tube and istalling a rod finished in an extension disc, with its corresponding ring seal, for the sealing of the inside of the tube; this way it is created a chamber, in the inside of the tube, in which the spring will exert a force opposing to the force exerted by the vacuum on the surface of the extension disc of the rod, producing an horizontal displacement of the rod proportional to the force constant of the spring and to the vacuum magnitude present in the tank; a graduated scale in the rod will serve as indicator of the vacuum magnitude. The operation as relief valve is obtained flowing air from the outer atmosphere to the tank across the tube (vacuum leak) by means of positioning of the ring seal of the rod extension disc in a location where is allowed this leak, this happens exclusively at the end of the path of the rod, in a place in where the compression of the spring implies a vacuum impossible to reach (-100kPa), to do not interfere in the functionality as vacuum sensor, that works in all the rest of the stroke; the relief valve is necessary to be able to use the apparatus in the application as auxiliary system of an obstetrical suction cup, in assistance to the birth, in which does not exist another way reduce the applied vacuum. In third and last place, the rod will also serve as blocking system of the pedal, thanks to having one end in hook form and to be positioned in an adequated place underneath the pedal.

A medical suction apparatus made in accordance with the invention has evident advantages with respect to the existing apparatuses, since it allows to fulfill all the objectives previously enumerated.

Next are described the constructive details of a preferred example for the present invention, and it is elucidated by reference to the embodiment partially illustrated schematically in the drawings:

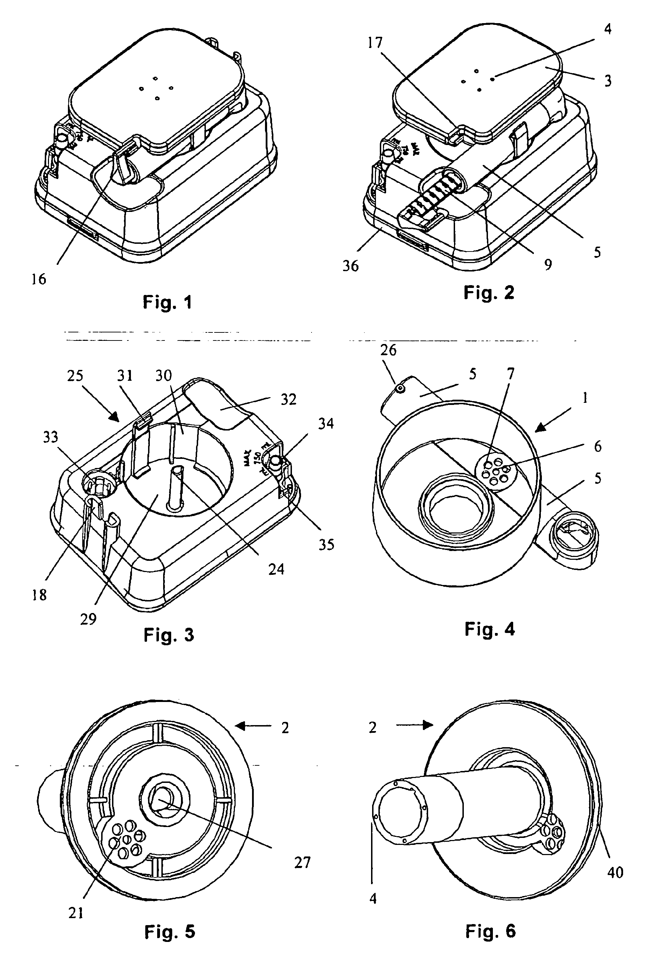

Fig 1 shows, in perspective, the assembly view with the apparatus fully assembled and the pedal in its blocking position by the sensor rod.
Fig 2 shows, in the same perspective that Fig 1, the assembly view with the apparatus fully assembled, and the pedal released and the sensor rod in its resting place.
Fig 3 shows, in perspective, the tank.
Fig 4 shows, in perspective, the cylinder.
Fig 5 shows, in perspective, the piston.
Fig 6 shows, in perspective, the piston turned 90° with respect to Fig 5.

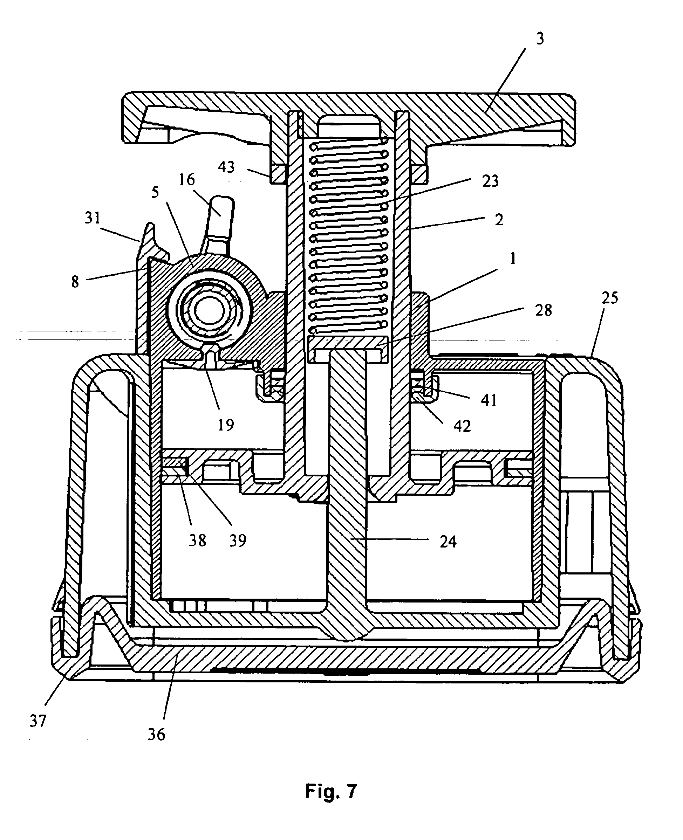

Fig 7 shows a front view of the assembly sectioned by the vertical plane passing through the axis of the piston and the axis of the suction valve; its object is to show the mechanism implied in the suction phase.

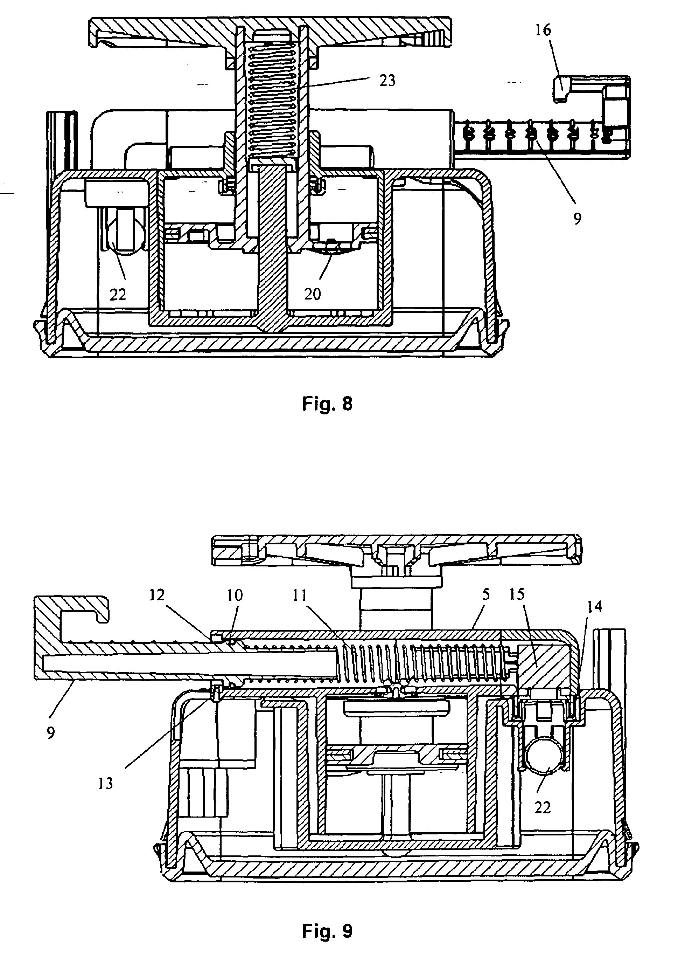

Fig 8 shows a front view of the assembly sectioned by the vertical plane passing through the axis of the piston and the axis of the expulsion valve; its object is to show the mechanism implied in the recovery phase.
Fig 9 shows a front view of the assembly sectioned by the vertical plane passing through the axis of the sensor rod; its object is to show the mechanism that combines vacuun sensor + relief valve + blocking system of the pedal, and to show to the link between the cylinder and the tank.

The piston (2) moves vertically through the chamber inside of the cylinder (1), driven directly by the pedal (3), this pedal is fixed to the piston by means of screws (4) or simply strongly fitted; in order to assure the watertightness of the inside chamber of the cylinder there are seals in the movable surfaces in contact: on the one hand the displacement of the base of the piston through the inside surface of the cylinder is made by means of two identical open segments (38) (39), installed in the lodging (40) of the base of the piston, and on the other hand the displacement of the axis of the piston through the surface of the neck of the cylinder is made by means of the ring seal (41) installed within the lodging formed by the neck of the cylinder and the necklace (42). The cylinder includes, located in its upper part, the sensor channel (5), this channel is a tube bent into an elbow, opened by its two ends, one horizontal and the other vertical downwards; the sensor channel (5) contains: in the center, internally the holes (6) of the suction valve (19), one of which also acts as leaking orifice (7) for the relief valve, and externally the top (8) for the clamp of the cylinder; in the horizontal section it is located the combined mechanism vacuum sensor + relief valve + blocking system of the pedal, formed by: the sensor rod (9), the ring seal of the rod (10), the spring of the rod (11), the top ring of the rod (12) and the fixing screw of the top ring of the rod (13) that lodges in its corresponding orifice (26) of the cylinder; finally, in the vertical section there are located: in the edge, the closing seal cylinder-tank (14), and in the inner the bacterial filter (15).
The sensor rod (9) has three different functions: in the first place it acts as indicator of the vacuum magnitude, allowing, thanks to its geometry in triangular prism, to indicate the vacuum in three different units; secondly it acts as blocking element of the pedal, thanks to the hook (16) that it has in his end and that lodges in a carved cavity (17) of the pedal (3); and thirdly it acts as relief valve, that works by pressing manually the rod until its end position, moment at which the ring seal (10) reaches to the first of the orifices (7) of the suction valve, allowing the air flow from the atmosphere to the tank throught the leak in the orifice, with the consequent progressive reduction of the vacuum.
The suction valve (19) is installed by introducing its rod in the central of the seven specific orifices (6) of the cylinder (1), with its lip towards the inner of the cylinder.
The expulsion valve (20) is installed by introducing its rod in the central of the seven specific orifices (21) of the piston (2), with its lip towards the outside of the cylinder.
The closing seal cylinder-tank (14) presents an additional lip in its inside orifice that will serve as closing seal for the floating ball (22).
The damping elastic ring (43) works to smooth the impact of the pedal (3) with the neck of the cylinder (1) at the end of the suction phase.
The recovery phase takes place thanks to the potential energy accumulated in the return spring (23), lodged in the inside of the hollow axis of the piston (2), and caused by the compression that in this spring produces the pushing rod (24), belonging to the tank (25), that is introduced in the inside of the hollow axis of the piston through the oval eccentric orifice (27) of its base, to push directly on the recovery piston (28); the recovery piston (28) crosses the hollow axis of the piston (2), towards the pedal in the suction phase and towards the base in the recovery phase, to transmit the force of the return spring (23) to the own piston by means of the top exerted by the pushing rod (24).
The tank (25) is a transparent piece to allow visualizing the level of the liquid inside of it, this piece must be made moulded due to it integrates multiple geometric details: firstly it contains a circular central draining (29) where the cylinder (1) will be anchoraged and clamped, in one of the laterals of such draining are carved some channels (30) to favor the free outlet of the air to the atmosphere during the cycling; secondly, in the center of the circular draining, it is located the pushing rod (24), whose section is oval, similar but smaller than as the one of the orifice (27) of the base of the piston, the objective of such geometry is to limit the rotation of the pedal (3) to avoid damages in the sensor rod (9) produced by the foot of the user; thirdly, the tank includes the clamp of the cylinder (31), destined to fit in the top (8) carved in the channel of the sensor (5) of the cylinder (1), this clamping will comfort hardly thanks to the pressure that exerts the return spring (23) in the cylinder (1); in fourth place it is the lateral carving (32) destined to allow the free turning of the sensor rod (9); in fifth place it is the lodging (33) of the vertical section of the sensor channel (5) of the cylinder, with his upper and lower tops to prevent that the floating ball (22) comes off the tank freely; in sixth place it is the lodging of the auxiliary bottle (18), whose female shaped geometry will allow to connect a collection bottle equipped with a compatible male connection; and in seventh, and last place, is the patient connection (34), located within a stepped form (35) designed to be able to represent and to emboss in the direction of molding of the piece, the level indicators of liquid.
The tank is closed by means of the cover (36), made in a slightly elastic material, of hardness between 70 and 85 shoreA, with almost flat geometry and a lip (37) in all its perimeter to allow the watertight closing with the tank; the cover serves in addition to nonskid horizontal support for the apparatus.

## Claims

1. Medical suction apparatus designed to be driven by the user by means of the foot or, failing that, with the hand; the apparatus contains a tank (25) that at the same times acts as liquids collection tank, serves as supporting frame for the apparatus and serves as anchorage for the cylinder-piston set; the apparatus works supported on a horizontal surface by driving a pedal (7) located in its upper part, that is firmly assembled to the axis of a piston of vertical displacement; interposed between the pedal and the piston there is a cylinder (1) assembled with its inside surface as stroke for the basis of the piston, wherein, for the operation of the apparatus it is necessary to anchor the cylinder in a a lodging (40) carved in the in the tank, the apparatus being **characterized in that** the axis of the piston is hollow, and there is a spring (23) lodged in the inside of this axis, that is responsable of the going up displacement of the piston - recovery phase -, and in addition the tank contains a vertical pushing rod (24) located within the lodging for the cylinder, whose function is to introduce itself in the inside of the hollow axis of the piston to exert on the spring the force that causes the going up movement.

2. Medical suction apparatus according to claim 1, **characterised in that** the vertical pushing rod (24) of the tank and the orifice of the base of the piston (27), have an asymmetric geometry with the intention of limiting the turn of the pedal respect to the cylinder.

3. Medical suction apparatus according to claim 1, **characterised in that** the cylinder integrates, in its same piece, a tube bent into an elbow (5), 90° downwards, communicated with the inside chamber of the cylinder by means of a set of orifices (6) located in its horizontal section and destined to install the suction valve (19), and whose vertical end, opened downwards, is assigned to be coupled, by means of a closing seal (14), in an specific orifice of the tank (33), thus to communicate the tank with the inside chamber of the cylinder during the suction phase.

4. Medical suction apparatus according to claim 3, **characterised in that** a filter of particles or bacteria (15) is located within the vertical section of the tube (5) and held by the closing seal (14) to prevent the coming out from its location.

5. Medical suction apparatus according to claim 3, **characterised in that** the horizontal section of the tube (5) is used to lodge a vacuum sensor, made up at least of a spring (11) and a rod (9) that moves throughout the horizontal axis of such piping a length proportional to the magnitude of the vacuum in the tank.

6. Medical suction apparatus according to claim 3, **characterised in that** the horizontal section of the tube (5) is used to lodge a relief valve, made up at least of a spring (11) and a rod (9) that allows that when pressing the rod takes place the air leak from the atmosphere to the tank.

7. Medical suction apparatus according to claim 1, **characterised in that** the cylinder integrates, in its same piece, a piping destined at least to lodge a rod that can turn on the piping axis, whose function is to hold the pedal in a blocking position, and with the object to allow the stocking of the apparatus in a configuration of minimum volume.

8. Medical suction apparatus according to claim 1, **characterised in that** the tank integrates, in its same piece, a clamp (31) as a system to anchor the cylinder-piston set on the tank, so that to release this set will be necessary to press the clamp in one direction, allowing then that the force of the spring (23) helps the cylinder-piston set moving out from its lodge.

9. Medical suction apparatus according to claim 1, **characterised in that** the tank integrates, in its same piece, a female profile connection dedicated to install a collection bottle equipped with a compatible male connection.

10. Medical suction apparatus according to claim 1, **characterised in that** the tank has a vertex in the shape of a staircase (35) that has represented or embossed, on each step, the volume corresponding to its level of liquid.

## Patentansprüche

1. Medizinisches Ansauggerät, das dazu konzipiert ist, von dem Benutzer mittels dem Fuß oder, falls dies fehlschlägt, von Hand betrieben zu werden; das Gerät umfasst einen Tank (25), der zur gleichen Zeit als Flüssigkeitssammeltank fungiert, als Stützrahmen für das Gerät dient und als Verankerung für den Zylinder-Kolben-Satz dient; das Gerät arbeitet gelagert auf einer horizontalen Oberfläche, indem ein in deren oberem Teil befindliches Pedal (7) angetrieben wird, das fest auf der Achse eines Kolbens mit vertikaler Bewegung montiert ist, zwischen dem Pedal und dem Kolben eingeschoben befindet sich ein Zylinder (1), der mit seiner inneren Oberfläche als Hub für die Basis des Kolbens montiert ist, worin es für den Betrieb des Geräts notwendig ist, den Zylinder in einer in den Tank geschnittenen Aufnahme (40) zu verankern, wobei das Gerät **dadurch gekennzeichnet ist, dass** die Achse des Kolbens hohl ist und eine Feder (23) in dem Inneren dieser Achse aufgenommen ist, die für die Aufwärtsbewegung des Kolbens - Rückgewinnungsphase - verantwortlich ist, und dass der Tank zusätzlich dazu eine vertikale treibende Kurbelstange (24) enthält, die sich innerhalb der Aufnahme für den Zylinder befindet, deren Funktion es ist, sich selbst in das Innere der hohlen Achse des Kolbens einzuführen, um auf die Feder die Kraft auszuüben, die die Aufwärtsbewegung verursacht.

2. Medizinisches Ansauggerät nach Anspruch 1, **dadurch gekennzeichnet, dass** die vertikale antreibende Kurbelstange (24) des Tanks und die Öffnung der Basis des Kolbens (27) eine asymmetrische Geometrie mit dem Ziel besitzen, die Drehung des Pedals gegenüber dem Zylinder zu begrenzen.

3. Medizinisches Ansauggerät nach Anspruch 1, **dadurch gekennzeichnet, dass** der Zylinder in seinem eigenen Teil ein in einem 90°-Krümmer (5) nach unten gebogenes Rohr integriert, das mittels eines Satzes von Öffnungen (6) mit der inneren Kammer des Zylinders verbunden ist, das sich in dessen horizontalem Querschnitt befindet und dazu bestimmt ist, das Ansaugventil (19) zu installieren, und dessen vertikales, nach unten geöffnetes Ende dazu bestimmt ist, mittels einer Verschlussdichtung (14) in einer spezifischen Öffnung des Tanks (33) gekoppelt zu werden, um so den Tank mit der inneren Kammer des Zylinders während der Ansaugphase zu verbinden.

4. Medizinisches Ansauggerät nach Anspruch 3, **dadurch gekennzeichnet, dass** sich ein Partikel- oder Bakterienfilter (15) innerhalb des vertikalen Querschnitts des Rohrs (5) befindet und von der Verschlussdichtung (14) gehalten wird, um das Herauskommen aus seiner Position zu verhindern.

5. Medizinisches Ansauggerät nach Anspruch 3, **dadurch gekennzeichnet, dass** der horizontale Querschnitt des Rohrs (5) dazu verwendet wird, um einen Vakuumsensor aufzunehmen, der mindestens aus einer Feder (11) und einer Kurbelstange (9) besteht, der sich durch die horizontale Achse einer solchen Leitung mit einer Länge proportional zur Größe des Vakuums in dem Tank bewegt.

6. Medizinisches Ansauggerät nach Anspruch 3, **dadurch gekennzeichnet, dass** der horizontale Querschnitt des Rohrs (5) dazu verwendet wird, um ein Ablassventil aufzunehmen, das mindestens aus einer Feder (11) und einer Kurbelstange (9) besteht, das erlaubt, dass bei Drücken der Kurbelstange der Lufteinlass aus der Atmosphäre an den Tank stattfindet.

7. Medizinisches Ansauggerät nach Anspruch 1, **dadurch gekennzeichnet, dass** der Zylinder in seinem eigenen Teil eine Rohrleitungen integriert, die dazu bestimmt sind, mindestens eine Kurbelstange aufzunehmen, die sich um die Rohrleitungsachse drehen kann, dessen Funktion es ist, das Pedal in einer Sperrposition zu halten, und mit dem Ziel, die Lagerung des Geräts in einer Konfiguration des geringsten Volumens zu erlauben.

8. Medizinisches Ansauggerät nach Anspruch 1, **dadurch gekennzeichnet, dass** der Tank in seinem eigenen Teil eine Klammer (31) als System integriert, um den Zylinder-Kolben-Satz auf dem Tank zu verankern, so dass es zur Auslösung dieses Satzes notwendig ist, die Klammer in eine Richtung zu drücken, wobei es dann erlaubt wird, dass die Kraft auf die Feder (23) es dem Zylinder-Kolben-Satz erleichtert, sich aus seiner Aufnahme heraus zu bewegen.

9. Medizinisches Ansauggerät nach Anspruch 1, **dadurch gekennzeichnet, dass** der Tank in seinem eigenen Teil eine Verbindung mit Buchsenprofil integriert, die dazu bestimmt ist, eine mit einer kompatiblen Steckerverbindung ausgestattete Sammelflasche zu installieren.

10. Medizinisches Ansauggerät nach Anspruch 1, **dadurch gekennzeichnet, dass** der Tank einen Knickpunkt in der Form einer Treppe (35) besitzt, bei der auf jeder Stufe das Volumen entsprechend dem Flüssigkeitsstand dargestellt oder eingeprägt ist.

## Revendications

1. Appareil d'aspiration médicale conçu pour être commandé par l'utilisateur à l'aide du pied ou, à défaut, de la main ; l'appareil comporte un réservoir (25) qui tout à la fois intervient en tant que réservoir collecteur des liquides, sert de monture de support pour l'appareil et sert d'ancrage pour l'ensemble piston-cylindre ; l'appareil fonctionne en appui sur une surface horizontale en commandant une pédale (7) située sur sa partie supérieure, qui est solidement assemblée à l'axe d'un piston à déplacement vertical ; placé entre la pédale et le piston, un cylindre (1) est assemblé avec sa surface interne comme course pour la base du piston, dans lequel, pour le fonctionnement de l'appareil, il est nécessaire d'ancrer le cylindre dans un logement (40) découpé dans le réservoir, l'appareil étant **caractérisé en ce que** l'axe du piston est creux, et **en ce qu'**il y a un ressort (23) logé à l'intérieur de cet axe, qui est responsable du mouvement de remontée du piston - phase de redressement -, et en outre le réservoir contient une tige-poussoir verticale (24) située à l'intérieur du logement pour le cylindre, dont la fonction est de s'introduire elle-même à l'intérieur de l'axe creux du piston pour exercer sur le ressort la force qui provoque le mouvement de remontée.

2. Appareil d'aspiration médicale selon la revendication 1, **caractérisé en ce que** la tige-poussoir verticale (24) du réservoir et l'orifice de la base du piston (27), ont une géométrie asymétrique dans le but de limiter le tour de la pédale par rapport au cylindre.

3. Appareil d'aspiration médicale selon la revendication 1, **caractérisé en ce que** le cylindre intègre, dans sa même pièce, un tube plié dans un coude (5), à 90° vers le bas, qui communique avec la chambre interne du cylindre au moyen d'un ensemble d'orifices (6) situés dans sa section horizontale et destinés à l'installation de la soupape d'aspiration (19), et dont l'extrémité verticale, ouverte vers le bas, est destinée à être couplée, au moyen d'un joint d'obturation (14), dans un orifice spécifique du réservoir (33), donc à faire communiquer le réservoir avec la chambre interne du cylindre durant la phase d'aspiration.

4. Appareil d'aspiration médicale selon la revendication 3, **caractérisé en ce qu'**un filtre à particules ou bactéries (15) est situé à l'intérieur de la section verticale du tube (5) et retenu par le joint d'obturation (14) pour empêcher qu'il ne sorte de son emplacement.

5. Appareil d'aspiration médicale selon la revendication 3, **caractérisé en ce que** la section horizontale du tube (5) est utilisée pour loger un capteur de vide, constitué au moins d'un ressort (11) et d'une tige (9) qui déplace tout le long de l'axe horizontal de ce tuyau une longueur proportionnelle à la grandeur du vide dans le réservoir.

6. Appareil d'aspiration médicale selon la revendication 3, **caractérisé en ce que** la section horizontale du tube (5) est utilisée pour loger une soupape de décharge, constitué au moins d'un ressort (11) et d'une tige (9) qui permet que, lorsqu'on comprime la tige, la fuite d'air de l'atmosphère jusqu'au réservoir se produise.

7. Appareil d'aspiration médicale selon la revendication 1, **caractérisé en ce que** le cylindre intègre, dans sa même pièce, un tuyau destiné au moins à loger une tige qui peut tourner sur l'axe du tuyau, dont la fonction est de retenir la pédale dans une position de blocage, et dans le but de permettre le stockage de l'appareil dans une configuration de volume minimal.

8. Appareil d'aspiration médicale selon la revendication 1, **caractérisé en ce que** le réservoir intègre, dans sa même pièce, une fixation (31) en tant que système d'ancrage de l'ensemble cylindre-piston sur le réservoir, de elle façon que pour libérer cet ensemble, il sera nécessaire de comprimer la fixation dans un sens, en permettant ensuite que la force du ressort (23) aide l'ensemble cylindre-piston à sortir de son logement.

9. Appareil d'aspiration médicale selon la revendication 1, **caractérisé en ce que** le réservoir intègre, dans sa même pièce, un raccord de profil femelle destiné à l'installation d'une bouteille collectrice équipée d'un raccord mâle compatible.

10. Appareil d'aspiration médicale selon la revendication 1, **caractérisé en ce que** le réservoir possède un sommet en forme d'escalier (35) sur lequel est représenté ou estampé, sur chaque marche, le volume correspondant à son niveau de liquide.
